# EUROPEAN PATENT APPLICATION

(11) **EP 2 153 862 A1**
(43) Date of publication of application: **17.02.2010**
(21) Application number: 08162391.0
(22) Date of filing: 14.08.2008
(51) Int. Cl.: A61M 25/10, A61M 29/00

(54) **Reverse Catheter**

(71) Applicant: V-Kardia Pty Ltd., Melbourne VIC 3004 (AU)
(72) Inventor: The designation of the inventor has not yet been filed
(74) Representative: Adamson Jones

(57) **Abstract**

A catheter (100) for conducting a fluid into or out of a blood vessel, the catheter including a body (102) having a proximal end, a closed distal end and a mid-longitudinal axis; and a balloon (104) attached to the body, the balloon having a proximal portion (124) and a distal portion (126). The body has a first lumen in communication with the balloon and a second lumen (114) adapted to convey a fluid to or from the blood vessel through a side opening (116) in the body, the side opening being located adjacent the proximal portion of the balloon.

## Description

### Field of the Invention

The present invention relates to improvements in devices designed to circulate a perfusate into and/or out of a portion of a body.

### Background of the Invention

Many therapeutic procedures can be performed percutaneously. Such procedures include investigative and diagnostic methods as well as medical and surgical treatments. Percutaneous techniques are advantageous because they are minimally invasive, and therefore present a reduced risk to the patient when compared with invasive surgeries requiring open access to tissues within the patient's body.

Catheters and sheaths are typically used to access internal sites by entering the patient's body through an incision or other entry point to the peripheral vasculature and navigating through the torturous paths of the vascular system to the target site. Tools, cameras illumination sources and transducers can be deployed through the catheters and sheaths enabling a physician to inspect the site, take biopsies, implant devices, repair damage and perform numerous other tasks often on an outpatient basis.

Minimally invasive percutaneous approaches have also presented an opportunity for localized delivery of therapeutic agents. Localization facilitates improved treatment since the agent may be delivered directly to a target site, (for example, an organ) thereby avoiding "first pass" degradation in the liver. The agent may therefore exhibit an improved therapeutic effect when compared with the same agent administered orally, intravenously or via the intramuscular route. Moreover, where the therapeutic agent has benefit to the target site but is likely to be toxic to other organs or tissues, localized delivery can minimize the exposure and hence the risk presented to cells in other parts of the body. An example is where a cytotoxic drug is to be delivered to a cancerous organ, but it is desired to minimize or prevent exposure of healthy organs to the drug.

Various devices and methods to facilitate the percutaneous delivery and collection of fluids to organs and tissues of the human body have been described in the art. Typically, these devices and methods include a first catheter to deliver a fluid to an organ or tissue and a second catheter to collect fluid exiting the organ or tissue. The collected fluid may be returned to the organ, or discarded as waste outside the body. These devices and methods have had varying degrees of success. Successful applications have been developed for use with cardiac tissue, but the conventional devices and techniques used for cardiac treatment are not always well suited for areas outside the heart. Some reasons for this are that vessels come in different sizes. Therefore an apparatus used in the heart is not generally suitable for applications outside the heart. Additionally, the pathways of the heart usually require special consideration, such as manoeuvrability around the arches of the coronary system.

Methods of catheter based isolated limb perfusion or infusion generally require standard catheters of greater than 50 cm length to be introduced from the contralateral femoral artery, across the aortic bifurcation and advanced to the common femoral artery (CFA). This ensures that the outlet of the catheter is proximal to the branch of the profunda femoris artery (PFA). In patients with PVD or an occluded superficial femoral vein, often the PFA provides the majority of blood flow to the lower limb. Therefore placing the outlet of the cannula in the CFA is ideal when the whole limb needs to be perfused.

Standard catheter introducers placed in the groin will have the outlet of the introducer in the superficial femoral artery (SFA), if inserted in an antegrade direction. Therefore, a standard balloon catheter would only deliver perfusate downstream of the SFA, which is not optimal. Accordingly, there exists a need to provide an improved device, system and method that in certain situations will permit more optimal delivery or drainage of a perfusate to a selected area of the body.

The reference to any prior art in this specification is not, and should not be taken as an acknowledgement or any form of suggestion that the referenced prior art forms part of the common general knowledge in Australia.

### Summary

The present invention in one preferred aspect provides for a catheter for conducting a fluid into or out of a blood vessel, the catheter comprising a body having a proximal end, a closed distal end and a mid-longitudinal axis; and a balloon attached to the body, the balloon having a proximal portion and a distal portion. The body has a first lumen in communication with the balloon and a second lumen adapted to convey a fluid to or from the blood vessel through a side opening in the body, the side opening being located adjacent the proximal portion of the balloon.

In another preferred aspect, the present invention provides for a system for conducting a fluid into or out of a blood vessel, the system comprising a catheter including a body having a proximal end, a distal end and a mid-longitudinal axis; a balloon attached to the body, the balloon having a proximal portion and a distal portion. The body has a first lumen in communication with the balloon, a second lumen having a length and an opening proximate the distal end, and a third lumen extending between a connection port and a side opening located adjacent the proximal portion of the balloon. The third lumen is adapted to convey a fluid to or from the port and the blood vessel through the side opening. The system further includes an instrument insertable through the second lumen, the instrument having a length greater than the length of the second lumen.

In another preferred aspect, the present invention provides a method for removing a perfusate from a patient, comprising placing a distal end of a catheter into a vein in an antegrade direction relative to the blood flow; diverting the blood flow from the vein through the catheter, the blood flow including the perfusate; and removing the perfusate from the patient using the catheter.

### Brief Description of the Figures

Fig. 1 is a partial perspective view of a reverse catheter in accordance with a preferred embodiment of the present invention.

Fig. 2 is a partial side elevation view of the catheter of Fig. 1.

Fig. 3 is a perspective view of the catheter of Fig. 1 shown inserted in an antegrade direction in a blood vessel.

Fig. 4 is a diagram showing a system utilising the catheter of Fig. 1 in accordance with another preferred embodiment of the present invention.

Fig. 5 is a perspective view of a reverse catheter in accordance with another preferred embodiment of the present invention.

Fig. 6 is a side elevation view of the catheter of Fig. 5.

Fig. 6A is a partial cross sectional side view of the catheter along line A-A of Fig. 6.

Figs. 7A to 7C show deployment of the catheter of Fig. 1 in accordance with a preferred embodiment of a method of the present invention.

Fig. 8 is a perspective view of the catheter of Fig. 1 shown inserted in a lower extremity vein, and the reverse catheter of Fig. 5 shown inserted a lower extremity artery.

Fig. 8A is an expanded view of the positioning of the catheter of Fig. 1 and the reverse catheter of Fig. 5 taken along line A-A of Fig. 8.

### Detailed Description of the Drawings

Alternative embodiments of the invention will be apparent to those skilled in the art from consideration of the specification and practice of the invention disclosed herein. It is intended that the specification and examples be considered as exemplary only, with a true scope and spirit of the invention being indicated by the claims which follow. Wherever possible, like numbers will refer to like parts.

As used herein, "fluid" is intended to mean a flowable material that may or may not include solid particles.

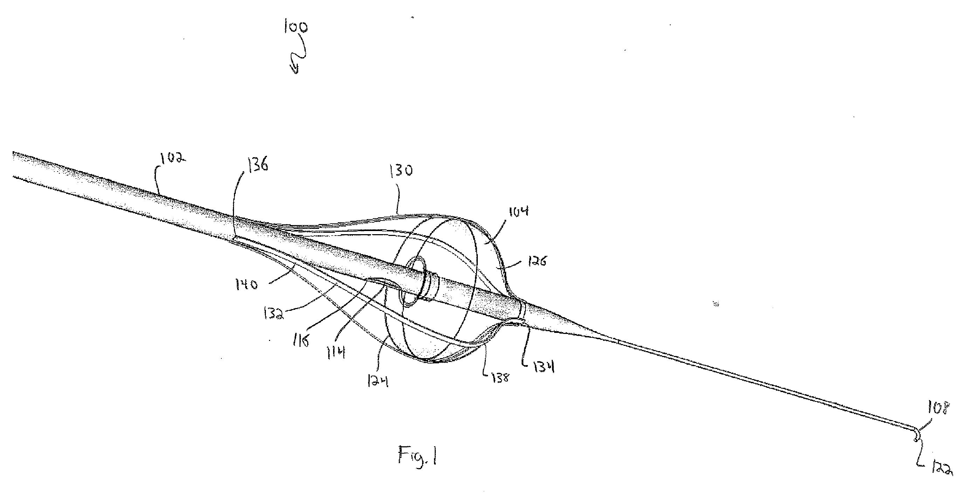

Figs. 1 to 3 show a preferred embodiment of a reverse catheter 100 having a body 102 and an occlusion means such as balloon 104. The preferred elements of catheter 100 and their interrelationship are described below.

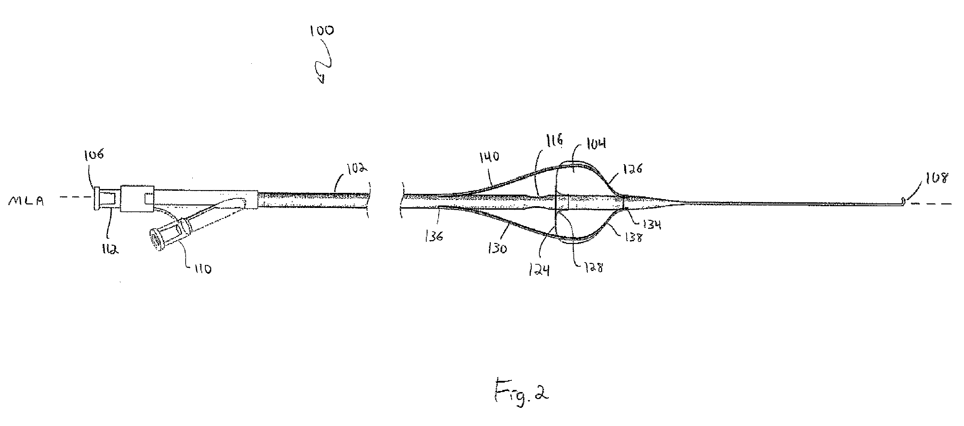

Referring to Figs. 1 and 2, body 102 includes a proximal end 106, a preferably closed distal end 108 and a mid-longitudinal axis MLA running through proximal end 106 and distal end 108. Proximal end 106 preferably includes a hub 110 configured as an inflation hub for balloon 104. Body 102 includes a lumen (not illustrated) extending between hub 110 and balloon 104 to inflate balloon 104. Body 102 preferably includes a second hub 112 adapted to connect to a source or repository of perfusion fluid. Body 102 includes a lumen 114 extending between hub 112 and a side opening 116 to convey perfusate between side opening 116 and hub 112. It will be appreciated that the reference to "side" is intended for easy reference and not limiting based on a particular position or orientation of the catheter in use.

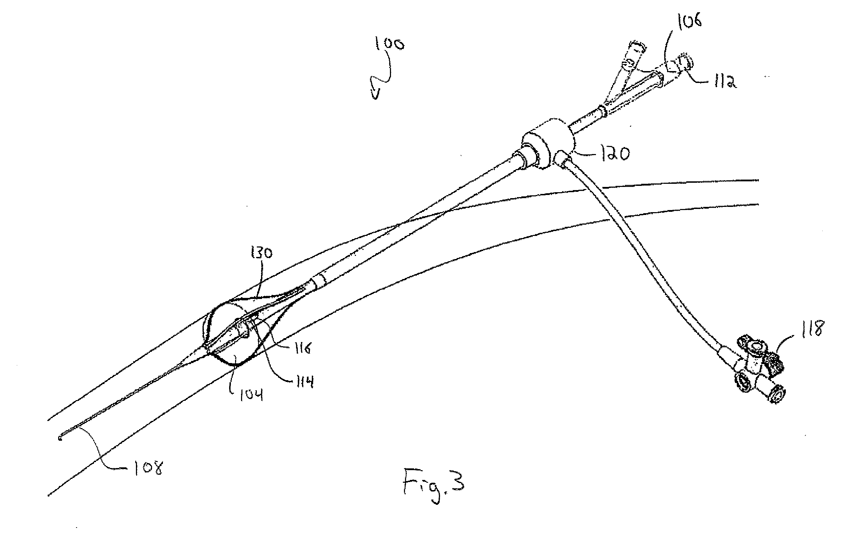

If desired, proximal end 106 may include hubs or ports for other uses. For example only, Fig. 3 shows a tap 118 connected to a hub 120 near proximal end 106.

As shown in Figs. 1 and 2, distal end 108 preferably includes an atraumatic tip 122 preferably configured as a J-tip or hook. It will be appreciated that tip 122 may be configured in a variety of ways without departing from the scope of the present invention. Examples of other tips are shown and described in International Application No. PCT/AU2006/001234 (Publication No. WO 2007/022592), the entire contents of which is hereby incorporated by reference herein.

Referring to Figs. 2 and 3, balloon 104 is preferably located closer to distal end 108 than proximal end 106. Balloon 104 includes a proximal portion 124 and a distal portion 126. Proximal portion 124 and distal portion 126 are preferably asymmetrically shaped relative to one another so that distal portion 126 generally forms a dome shape while proximal portion 124 generally forms a flattened end. Proximal portion 124 is preferably attached to body 102 so as to form a funnel region 128 relative to the exterior surface of body 102. Side opening 116 is preferably located adjacent proximal portion 124 so that perfusate delivery or collection occurs proximally relative to balloon 104.

As shown in Figs. 1 and 2, body 102 preferably includes an expandable support structure 130. Support structure 130 is preferably adapted to support and/or dilate a blood vessel into which catheter 100 is inserted. In particular, support structure 130 is beneficial in supporting the wall of the blood vessel against collapse when a negative pressure is present. Support structure 130 preferably includes a plurality of trusses 132 equally circumferentially spaced about the mid-longitudinal axis of body 102. Each of trusses 132 has a first end 134 and a second end 136 connected at different points along the length of body 102. Preferably, first end 134 is connected to body 102 distally of distal portion 126 of balloon 104, while second end 136 is connected proximally of side opening 116. Support structure 130 is preferably shaped so that a distal facing portion 138 generally forms a dome shape while a proximal facing portion 140 generally forms a cone shape with a gentle curve along a portion of its length as shown in Fig. 2. Examples of other forms of support structures are shown and described in International Application No. PCT/AU2005/000237 (Publication No. WO 2005/082440), the entire contents of which is hereby incorporated by reference herein.

The location of balloon 104 within support structure 130 beneficially permits the balloon to perform more than one function. For example, in addition to occluding the vessel, balloon is well placed to control trusses 132. When balloon 104 is deployed, the expansion of balloon 104 moves trusses 132 to deploy support structure 130. Support structure 130 may be configured as desired so that deflation of balloon 104 collapses support structure. Trusses 132 may be configured to be of sufficient resiliency and strength to support tissue without the aid of balloon 104 if desired.

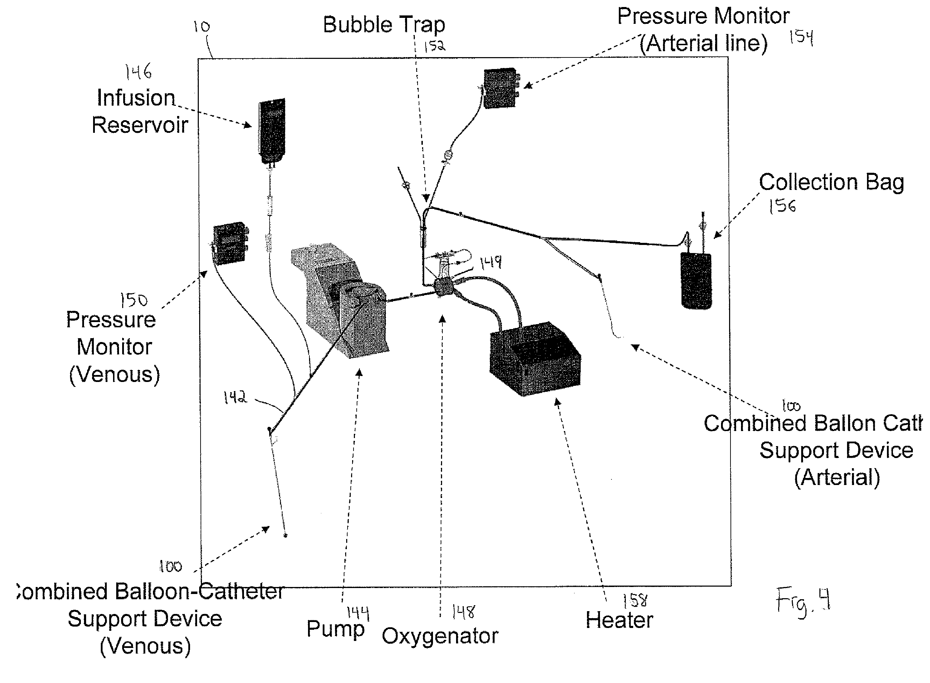

Fig. 4 shows an exemplary system 10 utilising catheter 100. System 10 preferably includes a venous collection catheter 100 connected to a tubing 142 at hub 112 (Fig. 2). Tubing 142 preferably runs through a peristaltic pump 144 configured to draw fluid into a perfusate reservoir 146 and/or oxygenator 148 (if the fluid is intended for re-use). Oxygenator preferably includes introducer ports 149 adapted for connection with other components either directly or indirectly via tubing. Pump 144 is preferably operationally connected to a pressure monitor 150 in order to permit the system to be automated. The system further preferably includes a pathway from pump 144 that leads to a bubble trap 152, a second pressure monitor 154, a collection bag 156 and/or delivery catheter 100. A heater 158 is preferably connected to oxygenator 148 to heat the fluid to a sufficient temperature for the intended purpose (e.g., introduction back into a patient). Various components of the system may preferably be in communication with a computer programmed to operate the system within specified parameters.

The catheter may be constructed from a variety of medically acceptable materials. For example, the trusses of support structure 130 may be made from Nitinol, or an elastic polymer (which may open up when the balloon is inflated).

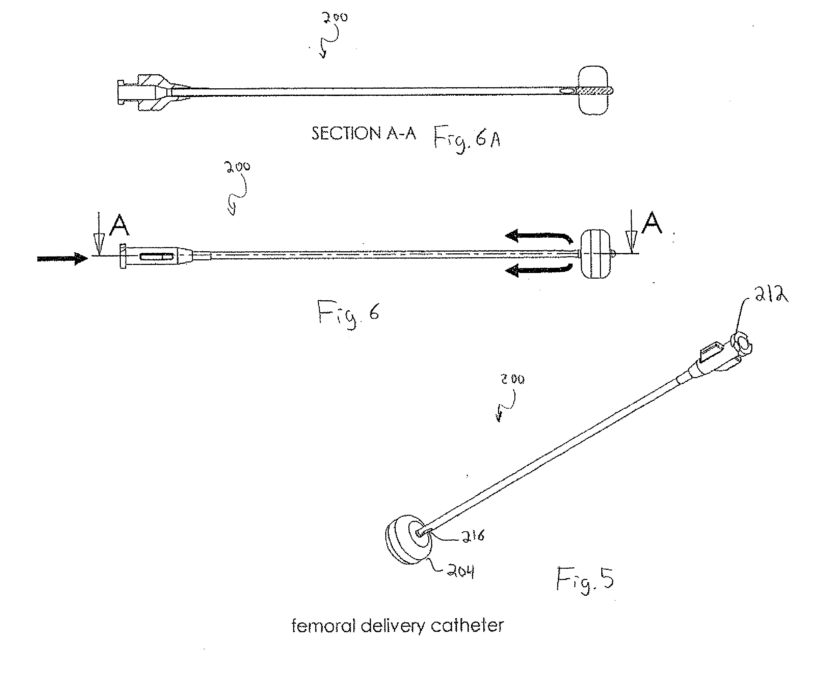

Referring now to Figs. 5 to 6A, a catheter 200 is shown in accordance with another preferred embodiment of the present invention. Catheter 200 is similar to catheter 100 except that the support structure is omitted around balloon 204. Additionally, the proximal and distal portions of balloon 204 are symmetrically shaped. Catheter 200 is preferably configured for use as a reverse delivery catheter and may be used in place of catheter 100 presently shown in Fig. 4. It will be appreciated that catheter 100 may also be configured as a reverse delivery catheter as desired.

A delivery device of the kind illustrated in Figs. 5 to 6A is suitable for delivering a blood (and therapeutic agent) solution from the artificial flow path to the targeted tissues. The blood/therapeutic agent solution may then be collected after passing through the target tissue using a venous collection device, such as catheter 100 in Fig. 2. Collected solution can then be re-circulated through the targeted tissue with re-oxygenated collected blood using a system such as system 10 in Fig. 4. Such a perfusion method is beneficial as it maximizes the efficiency of the therapeutic agent by eliminating break down in the liver and stomach, and also reduces or eliminates the toxicity issues associated with specific treatments reaching non-target tissue. Re-circulation further improves the uptake of therapeutic agents by increasing the exposure time to the target tissue, improving the effectiveness of treatment.

Having described the preferred components of catheter 100 and catheter 200, a preferred method of use will now be described with reference to Figs. 3, 5, and 7A to 8A. To perfuse a selected area of the body, the medical practitioner inserts catheter 200 into an artery (A) in a retrograde direction relative to the blood flow. Balloon 204 is deployed to occlude the pathway. The perfusate is introduced into the blood stream through hub 212, through a lumen and side opening 216 and into the blood vessel.

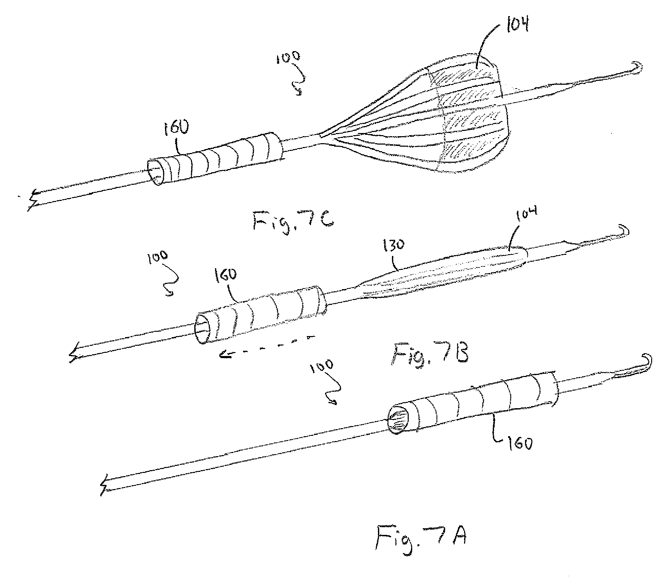

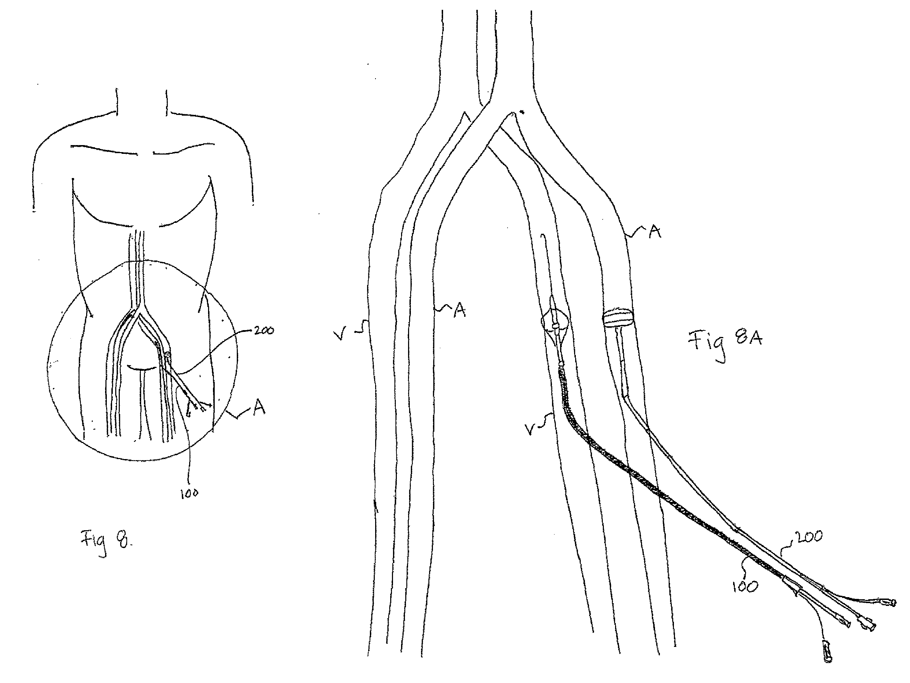

To collect the perfusate, the medical practitioner inserts catheter 100 into a vein (V) in an antegrade direction relative to the blood flow in an undeployed state, shown in Fig. 7A. A cover 160 is withdrawn to expose support structure 130 and balloon 104 as shown in Fig. 7B. Catheter 100 is moved to its deployed and engaged state as shown in Fig. 7C by deploying balloon 104 to occlude the pathway (see Fig. 8A). Support structure 130 may be deployed at the same time as balloon 104, or deployed prior to inflating the balloon as desired. The blood flow is diverted from the vein through side opening 116, lumen 114 and into a collection reservoir via hub 112 of catheter 100. Thereafter the blood containing the perfusate may be circulated into a delivery catheter for re-use as desired, or collected for later use or disposal. The perfusate being introduced into the delivery catheter may be replenished as needed and optionally supplemented by previously circulated perfusate. For example, system 10 shown in Fig. 4 may be used in performing the above method. Other exemplary circuits are described in International Application No. PCT/US2007/016369 (Publication No. WO 2008/011100), the entire contents of which is hereby incorporated by reference herein.

As shown in Fig. 8 and 8A, the catheters are preferably positioned at similar points in the respective artery and vein. It will be appreciated that the positioning may be varied as desired. Additionally, the above system and method may be used on only a portion of a limb if desired, for example, after an amputation.

Once the procedure has been substantially completed, balloon 104 is deflated, support structure 130 is collapsed and cover 160 is moved substantially back to its original position over support structure 130. It will be appreciated that the procedure may be performed without cover 160. Where support structure 130 is configured to spring outward, cover 160 may function to contain support structure 130 so that as cover 160 is withdrawn, support structure 130 deploys due to its own resiliency. Cover 160 may be used to collapse support structure 130 if desired. Cover 160 provides several advantages. For example, cover 160 protects the balloon and elements of the support structure during packaging and movement of the catheter. Cover 160 also protects vessels during insertion and removal of the catheter.

To perfuse a lower limb, catheter 100 is inserted into the vessel of the limb where perfusate is to be collected or delivered. By inserting the catheter in an antegrade direction in a vein, or a retrograde direction if inserting the catheter into an artery, the need to traverse the femoral archway (and the associated technical complexity of such a procedure) is eliminated. This has many advantages, such as use of catheters requiring less materials (and therefore costs), and a procedure that is less invasive.

It will be appreciated that the steps described above may be performed in a different order, varied, or certain steps omitted entirely without departing from the scope of the present invention. For example, catheter 100 may be inserted and withdrawn without the use of cover 160 if desired. Catheter 100 may be used as a reverse delivery catheter in lieu of catheter 200.

The foregoing description is by way of example only, and may be varied considerably without departing from the scope of the present invention. For example only, the catheter may include an open distal end in communication with the lumen conveying the perfusate, or in communication with a third lumen. The third lumen may be independent of the other lumens and adapted to receive therethrough a guide wire or other introducing instrument. One or more of the lumens may be concentric relative to the mid-longitudinal axis, or in a side-by-side relationship. One or more of the lumens may include one or more valves to assist in the control of the conveyance of the perfusate, and/or control of blood flow through the catheter. For example, where the catheter is adapted for use with a guide wire, a one-way valve may be included in the lumen containing the guide wire so that the valve may seal around the wire when the balloon is inflated (and before the pump is turned on).

In environments where a higher negative pressure might be needed, the catheter may be braded to enhance the structural integrity of the catheter.

The number, placement, shape and size of the side opening may be varied as desired. For example, side openings may be arranged equally spaced circumferentially around the mid-longitudinal axis of the body. Rows of openings may be arranged along the length of the body with varying sizes. For example, larger sized openings may be oriented closer to the balloon, while smaller sized openings are oriented further away from the balloon. The side openings may be shaped in a number of ways, for example, circular, oval, slit, or a grid. In one preferred embodiment, the side openings may be formed as a circumferential grid extending into the funnel region of the proximal portion of the balloon. This configuration is advantageous when the catheter is used, for example, as a drainage catheter. The shape of the balloon assists in diverting the flow of blood into the catheter.

Support structure 130 may be omitted if desired (as shown for example in Figs. 5 to 6A), or shaped in a variety of ways. For example, the trusses may each have a free end so as to be deployable similar to an umbrella, but with the free end being configured to reduce trauma (for example, being partially coiled or curved).

The catheter may include an external sleeve (such as cover 160 shown in Figs. 7A to 7C) or an internal rod or sheath to deploy the support structure manually. Preferably the side opening is located within the region between the proximal portion of the balloon and an end of the support structure to minimise interference of the blood vessel wall with the movement of fluid into or out of the side opening. For example, the side opening may be located a distance from the proximal portion of the balloon that is no greater than the maximum length of the balloon along the body.

Tip 120 may be internally guideable, or may be replaced with a guide wire as needed for a given situation.

Other instruments may be used in conjunction with the catheter as desired. For example, the catheter may be configured for use with a guide wire as described above, and/or with visualising instruments.

The device, system and method of the present invention may be applied to a variety of areas of the body, for example only, the lower extremeties such as the femoral arteries and/or veins, organs such as the liver, and other areas as desired. The present invention may be adapted for use in a variety of organs or anatomical regions. Exemplary organs include the lung, liver, kidney, brain, intestine, testicle, ovary, spleen, stomach, prostate, and pancreas. The anatomical region may be a limb, the pelvis, the chest, the breast or the mesenteric system.

Exemplary veins may include, but are not limited to a left or right internal jugular vein, a hepatic vein, a renal vein, a cephalic vein, an inferior vesical vein, a pulmonary vein, an internal iliac vein, a portal vein, a splenic vein, a femoral vein, a saphenous vein, a subclavian vein, an intercostal vein, an axillary vessel, a left or right testicular vein, a left or right ovarian vein, a pulmonary vein or tributaries thereof.

Exemplary arteries may include, but are not limited to a left or right vertebral artery, a left or right internal carotid artery, an inferior vesicular artery, an internal iliac artery, a renal artery, a gastric artery, a splenic artery, a superior or inferior mesenteric artery, an internal iliac artery, an internal mammary artery, an intercostal artery, a right or left testicular artery, a left or right ovarian artery, a pulmonary artery or tributaries thereof.

The method may further include the step of using an imaging technique to position the delivery catheter and/or collection catheter in the inflow and outflow vessels of the target region, the imaging technique including one or more of deployment of radiographic contrast, deployment of nuclear medical probes, deployment of in vivo probes sensitive to oxygen, hydrogen, pH or the like and deployment of labelled micro- or nano- particles. The imaging technique may be facilitated by including one or more materials in the tip of the catheter that are visible on the image produced by the imaging device. Ultrasound catheters may be used in lieu of, or in addition to the imaging techniques described above.

Embodiments of the present invention may be used in a range of blood vessels servicing different organs of the body including the lung, liver, kidney, brain, intestine, testicle, ovary, spleen, stomach, prostate and pancreas. The present invention may also be used in blood vessels servicing an entire anatomical region such as a limb, the pelvis, the chest, the breast and the mesenteric system. The skilled person will possess sufficient knowledge to decide the appropriate vessel or vessels to target according to which organ or anatomical region is to be catheterized. It is envisaged that the present invention has application beyond blood vessels, and may be used in other anatomical vessels or enclosed structures.

Where the organ is the liver, an arterial catheter may be placed in the hepatic artery and a venous catheter may be placed in the hepatic vein. This may be performed with a reduction of flow in the portal vein utilizing acute administration of, for example, Octreotide or Terlipressin.

Where the organ is the brain, arterial catheters may be placed in the left and right vertebral arteries, and also the left and right internal carotid arteries. Venous catheters may be placed in the left and right internal jugular veins.

Perfusion of the kidney may be performed by locating an arterial catheter in the renal artery. A venous catheter may be placed in the renal vein. Where the organ is the stomach, an arterial catheter may be placed in the gastric artery. A venous catheter may be placed in the portal vein or the hepatic veins.

Perfusion of the spleen may be achieved by placing an arterial catheter may in the splenic artery, and a venous catheter in the splenic vein, the portal vein, or the hepatic veins. To perfuse the intestines, an arterial catheter may be placed in the superior and/or inferior mesenteric artery. A venous catheter may be placed in the portal vein or hepatic veins.

Where the anatomical region is the pelvis, an arterial catheter may be placed in the internal iliac artery. Where the organ is a testicle or an ovary, an arterial catheter may be placed in the right or left testicular or ovarian artery. A venous catheter may be placed in the right or left testicular or ovarian vein. To perfuse the prostate, an arterial catheter may be placed in the inferior vesicle artery or the internal iliac artery. A venous catheter may be placed in the inferior vesical vein or the internal iliac vein.

Where the organ is a lung, an arterial catheter may be placed in the pulmonary artery. A venous catheter may be placed in one or both pulmonary veins. In one embodiment, selective delivery can be provided to one lung only, or only one lobe of a lung. Thus, the arterial catheter may be located in the relevant branch of the pulmonary artery and the venous catheter would be located in the relevant (i.e. upper or lower) pulmonary vein, depending on the lobe being treated. To perfuse the breast, an arterial catheter may be placed in the internal mammary artery and the intercostal arteries. A venous catheter may be placed in the intercostal veins.

It will be appreciated that other occlusion means may be used in addition to or in lieu of a balloon. For example, a suitably configured catheter may be inserted as described above after clamping one or more portions of the blood vessel instead of, or in addition to deploying a balloon. A tourniquet may be applied for occlusion in addition to or in lieu of a balloon as desired.

The features described with respect to one embodiment may be applied to other embodiments, or combined with or interchanged with the features other embodiments, as appropriate, without departing from the scope of the present invention.

The present invention in one or more preferred forms provides the advantages of being shorter and able to withstand higher flow rates than a standard length catheter. Therefore the catheter diameter may be reduced to be less invasive. A catheter of reduced length is easier to insert and control when compared to conventional length catheters. A further advantage is that in a preferred method of the present invention, for example, in delivering perfusate, the need to cross the aortic bifurcation is eliminated, reducing the length of the procedure.

In a preferred form, the supporting structure is configured to substantially prevent collapse of the blood vessel or other tissue under negative pressure conditions. The maintenance of the vessel enables an artificial circuit to be established, which may be automated with the use of one or more appropriately placed detectors and pumps.

The system and method described has wide applicability and may be adapted for use in animals other than humans, such as horses, dogs, cats, bulls, sheep and other sporting animals, domestic pets and/or livestock.

It will of course be realised that the above has been given only by way of illustrative example of the invention and that all such modifications and variations thereto as would be apparent to persons skilled in the art are deemed to fall within the broad scope and ambit of the invention as herein set forth.

## Claims

1. A catheter for conducting a fluid into or out of a blood vessel, said catheter comprising:
a body having a proximal end, a closed distal end and a mid-longitudinal axis; and
a balloon attached to said body, said balloon having a proximal portion and a distal portion, said body having a first lumen in communication with said balloon and a second lumen adapted to convey a fluid to or from the blood vessel through a side opening in said body, said side opening being located adjacent said proximal portion of said balloon.

2. The catheter of claim 1, wherein said first and second lumens are oriented concentric relative to one another.

3. The catheter of claim 1, wherein said first and second lumens are oriented in a side-by-side arrangement relative to one another.

4. The catheter of any one of the preceding claims, further comprising an expandable support structure adapted to support the blood vessel.

5. The catheter of claim 4, wherein said support structure includes a plurality of trusses, each of said trusses having two ends connected at different points along said body.

6. The catheter of claim 5, wherein said trusses extend over said balloon along the length of said balloon.

7. The catheter of either claim 5 or 6, wherein said side opening is located between said proximal portion of said balloon and one of the ends of said trusses.

8. The catheter of any one of claims 1 to 6, wherein said side opening is located a distance from said proximal portion of the balloon that is no greater than the maximum length of said balloon along said body.

9. A system for conducting a fluid into or out of a blood vessel, said system comprising:
a catheter including:
a body having a proximal end, a distal end and a mid-longitudinal axis;
a balloon attached to said body, said balloon having a proximal portion and a distal portion, said body having a first lumen in communication with said balloon, a second lumen having a length and an opening proximate said distal end, and a third lumen extending between a connection port and a side opening located adjacent said proximal portion of said balloon, said third lumen being adapted to convey a fluid to or from the port and the blood vessel through said side opening; and
an instrument insertable through said second lumen, said instrument having a length greater than the length of said second lumen.

10. The system of claim 9, wherein said first, second and third lumens are oriented concentric relative to one another.

11. The system of claim 9, wherein at least two of said lumens are oriented in a side-by-side arrangement relative to one another.

12. The system of any one of claims 9 to 11, said catheter further comprising an expandable support structure adapted to support the blood vessel.

13. The system of claim 12, wherein said support structure includes a plurality of trusses, each of said trusses having two ends connected at different points along said body.

14. The system of claim 13, wherein said trusses extend over said balloon along the length of said balloon.

15. The system of either claim 13 or 14, wherein said side opening is located between said proximal portion of said balloon and one of the ends of said trusses.
